# EUROPEAN PATENT APPLICATION

(11) **EP 3 047 917 A1**
(43) Date of publication of application: **27.07.2016**
(21) Application number: 15181653.5
(22) Date of filing: 20.08.2015
(51) Int. Cl.: B06B 1/06, G10K 11/02

(54) **MATCHING MEMBER AND ULTRASOUND PROBE INCLUDING THE SAME**

(30) Priority: 12.01.2015 KR 20150004447
(71) Applicant: Samsung Medison Co., Ltd., Hongcheon-gun, Gangwon-do 25108 (KR)
(72) Inventor: Lee, Won-hee, Gangwon-do (KR); Nam, Gi-ung, Gangwon-do (KR); Son, Kwang-jin, Gangwon-do (KR); Song, Kyung-hun, Gangwon-do (KR)
(74) Representative: Frey, Sven Holger

(57) **Abstract**

Provided are a matching member and an ultrasound probe including the matching member. The ultrasound probe includes a piezoelectric layer for converting an ultrasound wave to an electric signal or an electric signal to an ultrasound wave; and a matching layer disposed on the piezoelectric layer and having a particle-in-binder (PIB) structure in which a plurality of conductive particles and a binder are mixed, wherein at least one of the plurality of conductive particles includes: a core; and a shield formed of a different material than that of the core and surrounding a surface of the core.

## Description

### RELATED APPLICATION

This application claims the benefit of Korean Patent Application No. 10-2015-0004447, filed on January 12, 2015, in the Korean Intellectual Property Office, the disclosure of which is incorporated herein in its entirety by reference.

### BACKGROUND

### 1. Field

One or more exemplary embodiments relate to a conductive matching member and an ultrasound probe including the conductive matching member.

### 2. Description of the Related Art

In general, ultrasound diagnosis apparatuses transmit ultrasound waves to an object such as a human being or an animal and detect an echo signal reflected by the object to display a cross-sectional image of organs on a monitor and provide information necessary to diagnose the object. Here, ultrasound diagnosis apparatuses include an ultrasound probe for transmitting the ultrasound wave into the object and receiving the echo signal from the object.

In addition, an ultrasound probe includes a piezoelectric layer disposed therein to convert ultrasound signals and electric signals into each other, and the piezoelectric layer generally includes an assembly of a plurality of piezoelectric members. Therefore, an ultrasound diagnosis apparatus including such above members irradiates ultrasound waves to a target object and converts an echo signal of the ultrasound wave into an electric signal to generate an ultrasound image.

An ultrasound diagnosis apparatus using such above ultrasound probe is widely used for medical usage, for example, detection of impurities in a living body, measuring wounds, observing a tumor, and observing an embryo.

Research into a method of increasing a degree of freedom in designing the ultrasound probe has been conducted.

### SUMMARY

One or more embodiments include a matching member having electric conductivity and an ultrasound probe including the matching member.

Additional aspects will be set forth in part in the description which follows and, in part, will be apparent from the description, or may be learned by practice of the presented embodiments.

According to one or more embodiments, an ultrasound probe includes: a piezoelectric layer for converting an ultrasound wave to an electric signal or vice versa; and a matching layer disposed on the piezoelectric layer and having a particle-in-binder (PIB) structure in which a plurality of conductive particles and a binder are mixed, wherein at least one of the plurality of conductive particles includes: a core; and a shield formed of a different material than that of the core and surrounding a surface of the core.

An electric conductivity of the core may be different from an electric conductivity of the shield.

The electric conductivity of the shield may be greater than the electric conductivity of the core.

The shield may be formed of at least one selected from silver (Ag), gold (Au), and platinum (Pt).

The core may be formed of at least one selected from a non-conductive material, a semiconductor material, and a conductive material.

The core may be formed of at least one selected from copper (Cu), aluminum (Al), nickel (Ni), tungsten (W), beryllium (Be), indium (In), iron (Fe), lead (Pb), titanium (Ti), tin (Sn), glass, and silicon (Si).

At least one of the plurality of conductive particles may be formed as at least one selected from a sphere, a flake, a bar, a rod, a wire, a fiber, and a cone.

Some neighboring conductive particles among the plurality of conductive particles at least partially may overlap each other.

The plurality of conductive particles may include a first conductive particle and a second conductive particle, wherein an electric conductivity of the first conductive particle may be different from that of second conductive particle.

The first conductive particle may be different from the second conductive particle in at least one of a size and a material.

A material forming a core of the first conductive particle may be different from a material forming a core of the second conductive particle.

A composition ratio of the first conductive particle and the second conductive particle may vary in a thickness direction of the matching layer.

A content amount of the plurality of conductive particles in the matching layer may vary in a thickness direction of the matching layer.

The matching layer may be grounded.

The ultrasound probe may further include a ground electrode contacting the matching layer.

According to one or more embodiments, a matching member for matching acoustic impedances, the matching member includes a particle-in-binder (PIB) structure, in which a plurality of particles and a binder are mixed, wherein at least one of the plurality of conductive particles includes: a core; and a shield formed of a different material than that of the core and surrounding a surface of the core.

An electric conductivity of the shield may be greater than an electric conductivity of the core.

The core may be formed of at least one of selected from a non-conductive material, a semiconductor material, and a conductive material, and the shield is formed of at least one of selected from silver (Ag), gold (Au), and platinum (Pt).

Some neighboring conductive particles among the plurality of conductive particles at least partially may overlap with each other.

According to one or more embodiments, a matching member for matching acoustic impedances, the matching member includes a structure including a foam-type body that is porous and a filler filled in the foam-type body, wherein the foam-type body includes: a core; and a shield formed of a different material than that of the core and surrounding a surface of the core.

### BRIEF DESCRIPTION OF THE DRAWINGS

These and/or other aspects will become apparent and more readily appreciated from the following description of the embodiments, taken in conjunction with the accompanying drawings in which:
FIG. 1 is a block diagram of an ultrasound diagnosis apparatus according to an embodiment of the inventive concept;
FIG. 2 is a block diagram of an ultrasound probe of FIG. 1;
FIG. 3 is a schematic diagram of a physical configuration of the ultrasound probe of FIG. 2;
FIGS. 4A and 4B are diagrams showing states of arranging piezoelectric members in a piezoelectric layer according to an embodiment of the inventive concept;
FIG. 5 is a diagram of a matching layer according to an embodiment of the inventive concept;
FIG. 6 is a table illustrating sound characteristic results of a matching layer including a particle-in-binder (PIB) structure, according to an embodiment of the inventive concept;
FIGS. 7A to 7D are diagrams exemplarily showing matching layers including conductive particles according to embodiments of the inventive concept;
FIG. 8 is a diagram of a matching layer for adjusting an acoustic impedance by using one layer, according to an embodiment of the inventive concept; and
FIG. 9 is a diagram of a matching layer according to another embodiment of the inventive concept.

### DETAILED DESCRIPTION

Reference will now be made in detail to embodiments, examples of which are illustrated in the accompanying drawings, wherein like reference numerals refer to like elements throughout. As used herein, the term "and/or" includes any and all combinations of one or more of the associated listed items. Expressions such as "at least one of," when preceding a list of elements, modify the entire list of elements and do not modify the individual elements of the list.

Throughout the specification, an "object" may be a human, an animal, or a part of a human or animal. For example, the object may be an organ (e.g., the liver, heart, womb, brain, breast, or abdomen), a blood vessel, or a combination thereof. Throughout the specification, a "user" may be, but is not limited to, a medical expert including a medical doctor, a nurse, a medical laboratory technologist, a medial image expert, or a technician who repairs a medical apparatus.

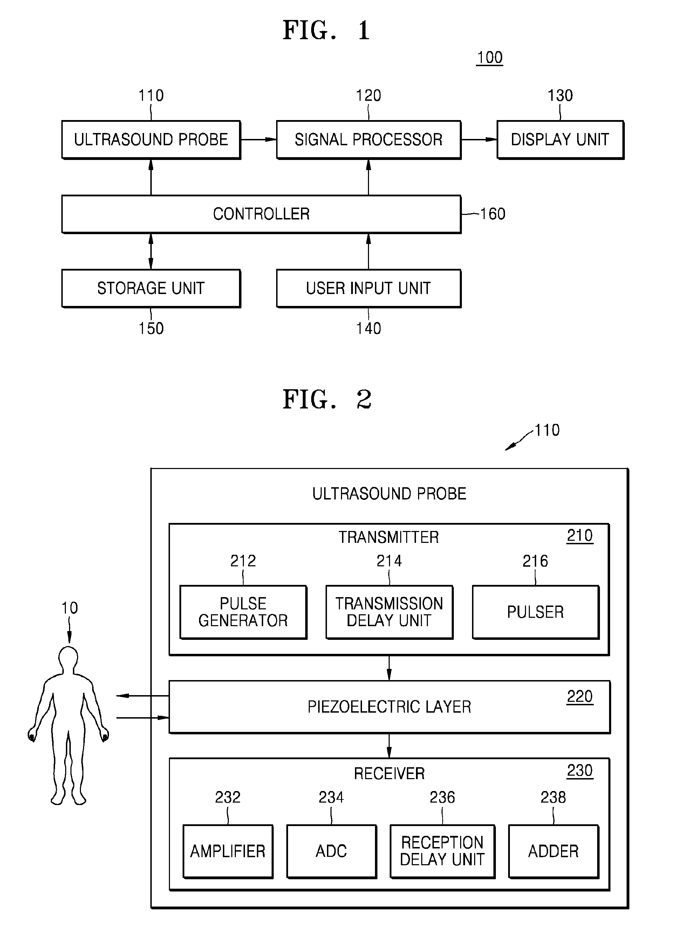

FIG. 1 is a block diagram of an ultrasound diagnosis apparatus 100 according to an exemplary embodiment of the inventive concept. Referring to FIG. 1, the ultrasound diagnosis apparatus 100 includes an ultrasound probe 110 for transmitting and receiving ultrasound waves, a signal processor 120 processing a signal applied from the ultrasound probe 110 to generate an image, a display unit 130 for displaying the image, a user input unit 140 for receiving a user input, a storage unit 150 storing various pieces of information, and a controller 160 controlling overall operations of the ultrasound diagnosis apparatus 100.

The ultrasound probe 110 is an apparatus for transmitting an ultrasound wave to an object and receiving an echo signal of the ultrasound wave reflected by the object, as described in more detail later.

The signal processor 120 processes ultrasound data generated by the ultrasound probe 110 to generate an ultrasound image. The ultrasound image may be at least one selected from a brightness mode (B mode) image representing the magnitude of an ultrasound echo signal reflected by an object as brightness, a Doppler mode (D mode) image representing an image of a moving object as a spectrum by using a Doppler effect, a motion mode (M mode) image representing movement of an object at a constant location according to time, an elastic mode image representing a difference between reactions when compression is applied and not applied to an object as an image, and a color mode (C mode) image representing a velocity of a moving object as a color by using a Doppler effect. Since the ultrasound image is generated by using an ultrasound image generating method that is currently implemented, detailed descriptions thereof are omitted here. Accordingly, the ultrasound image may be a one-dimensional (1 D) image, a two-dimensional (2D) image, a three-dimensional (3D) image, or a four-dimensional (4D) image.

The display unit 130 displays information processed by the ultrasound diagnosis apparatus 100. For example, the display unit 130 may display the ultrasound image generated by the signal processor 120, or may display a graphical user interface (GUI) for requesting a user input.

The display unit 130 may include at least one selected from a liquid crystal display, a thin film transistor-liquid crystal display, an organic light-emitting diode display, a flexible display, a 3D display, and an electrophoretic display, and the ultrasound diagnosis apparatus 100 may include two or more display units 130.

The user input unit 140 is a unit, to which a user inputs data to control the ultrasound diagnosis apparatus 100. The user input unit 140 may include a keypad, a mouse, a touch panel, or a track ball. The user input unit 140 is not limited to the above examples, and may further include various input units such as a jog wheel or a jog switch.

In addition, the touch panel may detect a proximity touch, that is, a case where a pointer approaches a screen within a predetermined distance, as well as a real touch, that is, a case where the pointer actually touches the screen. In the present specification, the pointer is a tool for touching or proximity touching a certain point of the touch panel, for example, a stylus pen or a body part such as a finger.

Also, the touch panel may be realized as a touch screen forming a layer structure with the display unit 130, and may be a capacitive overlay type, a resistive overlay type, an infrared beam type, a surface acoustic wave type, an integral strain gauge type, and a piezo electric type. The touch screen may function as the user input unit 140, as well as the display unit 130, and thus, may be widely used.

Although not shown in FIG. 1, the touch panel may include various sensors in or around the touch panel in order to sense a touch input. An example of the sensors for sensing the touch input may be a tactile sensor. The tactile sensor senses a contact of a certain material at an intensity that a human being may feel or greater. The tactile sensor may sense various pieces of information such as the roughness of a contacting surface, the solidity of a contact material, and the temperature at a contact point.

Also, an example of the sensor for sensing the touch on the touch screen may be a proximity sensor. The proximity sensor is a sensor for detecting whether an object approaches a predetermined detection surface or whether the external object is present nearby by using a force of an electromagnetic field or an infrared ray without an actual physical touch. Examples of the proximity sensor include a transparent photoelectric sensor, a direct reflective photoelectric sensor, a mirror reflective photoelectric sensor, a high frequency oscillation photoelectric sensor, a capacitive photoelectric sensor, a magnetic photoelectric sensor, an infrared photoelectric sensor, etc.

The storage unit 150 stores various pieces of information processed by the ultrasound diagnosis apparatus 100. For example, the storage unit 150 may store medical data regarding diagnosis of an object, for example, images, and may store algorithms or programs executed in the ultrasound diagnosis apparatus 100.

The storage unit 150 may include at least one type of a storage medium selected from a flash memory type, a hard disk type, a multimedia card micro type, a card type memory (for example, SD, XD memory, etc.), random access memory (RAM), static random access memory (SRAM), read only memory (ROM), electrically erasable programmable read-only memory (EEPROM), programmable read-only memory (PROM), a magnetic memory, a magnetic disk, and an optical disk. Also, the ultrasound diagnosis apparatus 100 may use a web storage or a cloud server performing a storage function of the storage unit 150 on the Internet.

The controller 160 controls overall operations of the ultrasound diagnosis apparatus 100. That is, the controller 160 may control operations of the ultrasound probe 110, the signal processor 120, and the display unit 130 shown in FIG. 1. For example, the controller 160 may control the signal processor 120 to generate an image by using a user input that is performed via the user input unit 140 or a program stored in the storage unit 150. Also, the controller 160 may control the display unit 130 to display the image generated by the signal processor 120.

FIG. 2 is a block diagram of the ultrasound probe 110 of FIG. 1. Referring to FIG. 2, the ultrasound probe 110 is a device for transmitting an ultrasound wave to an object 10 and receiving an echo signal reflected by the object 10 to generate ultrasound data. The ultrasound probe 110 may include a transmitter 210, a piezoelectric layer 220, and a receiver 230.

The transmitter 210 supplies a driving signal to the piezoelectric layer 220. The transmitter 210 may include a pulse generator 212, a transmission delay unit 214, and a pulser 216.

The pulse generator 212 generates rate pulses for forming a transmission frequency according to a predetermined pulse repetition frequency (PRF). The transmission delay unit 214 applies a delay time for determining transmission directionality to the rate pulses generated by the pulse generator 212. The rate pulses, to which the delay time is applied, respectively correspond to a plurality of piezoelectric members 222 included in the piezoelectric layer 220. The pulser 216 applies driving signals (or driving pulses) to the piezoelectric layer 220 at timings corresponding respectively to the rate pulses, to which the delay time is applied.

The piezoelectric layer 220 transmits the ultrasound wave to the object 10 according to the driving signal supplied from the transmitter 210, and receives an echo signal of the ultrasound wave reflected by the object 10. The piezoelectric layer 220 may include the plurality of piezoelectric members 222 that convert an electric signal to acoustic energy (or vice versa).

The receiver 230 processes a signal transmitted to the piezoelectric layer 220 to generate ultrasound data. The receiver 230 may include an amplifier 232, an analog digital converter (ADC) 234, a reception delay unit 236, and an adder 238.

The amplifier 232 amplifies the signal transmitted from the piezoelectric layer 220, and the ADC 234 performs analog-digital conversion of the amplified signal. The reception delay unit 236 applies a delay time for determining the reception directionality to the digitally converted signal. The adder 238 adds up the signals processed by the reception delay unit 236 to generate the ultrasound data. A component reflected from a direction determined by the reception directionality may be emphasized by the adding process of the adder 238.

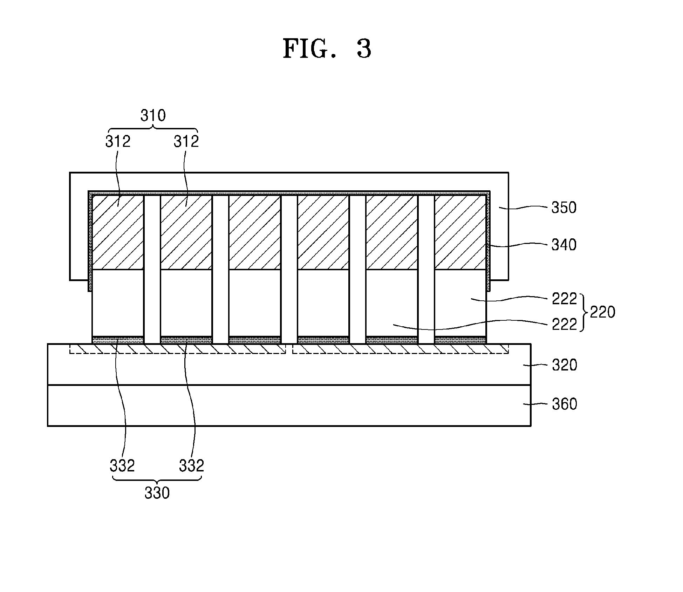

The transmitter 210 and the receiver 230 of the ultrasound probe 110 may be formed as at least a chip on a substrate 240 (see FIG. 3). Here, the substrate 240 may be formed of silicon (Si), ceramic, or a polymer-based material. Otherwise, the substrate 240 may be formed of a backing material for absorbing ultrasound waves. Each of the blocks in the transmitter 210 and the receiver 230 may be formed as a chip, or two or more blocks may be formed as a chip. In addition, a chip may be formed to correspond to one piezoelectric member 222. Thus, the substrate including at least one of the transmitter 210 and the receiver 230 is referred to as a chip module substrate. The chip module substrate may denote a substrate including some of the chips included in the ultrasound probe 110, as well as a substrate including all of the chips included in the ultrasound probe 110.

In addition, the ultrasound probe 110 may further include some components of the signal processor 120, some components of the display unit 130, and some components of the user input unit 140, in addition to the transmitter 210 and the receiver 230.

FIG. 3 is a schematic diagram showing a physical configuration of the ultrasound probe 110 of FIG. 2. As shown in FIG. 3, the ultrasound probe 110 may include the piezoelectric layer 220 for converting the ultrasound wave to the electric signal (or vice versa), and a matching layer 310 for matching an acoustic impedance of the ultrasound wave generated from the piezoelectric layer 220 to an acoustic impedance of the object 10.

The ultrasound probe 110 may further include a chip module substrate 320 including at least one chip for processing electric signals, a first electrode 330 disposed between the piezoelectric layer 220 and the chip module substrate 320 to electrically connect the piezoelectric layer 220 and the chip module substrate 320 to each other, and a second electrode 340 disposed on the matching layer 310 to electrically connect the piezoelectric layer 220 and the chip module substrate 320 to each other.

The piezoelectric layer 220 may include a plurality of piezoelectric members 222 that convert an electric signal to acoustic energy (or vice versa). The plurality of piezoelectric members 222 may be spaced apart from each other. Each of the piezoelectric members 222 may be formed of a material causing a piezoelectric phenomenon, for example, at least one selected from ZnO, AlN, PbZrTiO₃ (PZT), PbLaZrTiO₃ (PLZT), BaTiO₃ (BT), PbTiO₃ (PT), and Pb(Mg_{1/3}Nb_{2/3})O₃-PbTiO₃ (PMN-PT).

The matching layer 310 is disposed on a front surface of the piezoelectric layer 220, and gradually changes the acoustic impedance of the ultrasound wave generated from the piezoelectric layer 220 so as to make the acoustic impedance of the ultrasound wave similar to the acoustic impedance of the object 10. The acoustic impedance of the matching layer 310 is greater than that of the piezoelectric layer 220 and less than that of the object 10. Here, the front surface of the piezoelectric layer 220 may denote a surface that is the closest to the object 10 while the ultrasound wave is emitted to the object 10, and a rear surface may be an opposite surface of the front surface.

The matching layer 310 may also include a plurality of matching members 312 disposed respectively on the piezoelectric members 222. However, one or more exemplary embodiments are not limited thereto, and, one matching member 312 of the matching layer 310 may correspond to a group of piezoelectric members 222. The matching layer 310 may have a single-layered structure or a multi-layered structure.

The matching layer 310 according to the embodiment may have electric conductivity, as well as matching of the acoustic impedance. The matching layer 310 may have a particle-in-binder (PIB), in which a plurality of conductive particles 510 and a binder 520 are mixed. The structure of the matching layer 310 will be described later.

The chip module substrate 320 is a substrate including at least one chip for processing the electric signal, as described above. For example, at least one chip performing functions of the receiver 230 and the transmitter 210 may be disposed on the chip module substrate 320. The chip module substrate 320 may be an application specific integrated circuit (ASIC), but is not limited thereto.

The first electrode 330 electrically connects the piezoelectric layer 220 to the chip module substrate 320. The first electrode 330 may include a plurality of electrode members 332 that connect the piezoelectric members 222 respectively to the chip module substrate 320 and are spaced apart from each other. Each of the electrode members 332 may electrically connect one piezoelectric member 222 to the chip module substrate 320. In addition, the electrode members 332 may be formed of a conductive material. The driving signal may be applied to the piezoelectric layer 220 via the first electrode 330.

The second electrode 340 also electrically connects the piezoelectric layer 220 to the chip module substrate 320. The second electrode 340 may be formed as a layer on the piezoelectric layer 220. The second electrode 340 may include a conductive material. A ground signal may be applied to the piezoelectric layer 220 via the second electrode 340. In addition, since the second electrode 340 is formed of a conductive material, the second electrode 340 may remove noise caused by a radio frequency induced from outside. Moreover, the second electrode 340 may further include a material for blocking chemical impurities introduced from outside. For example, the second electrode 340 may include polyimide.

The second electrode 340 may be formed as a single layer, not as a plurality of members to correspond to the piezoelectric members 222. That is, the second electrode 340 may not be formed of a plurality of members that are spaced apart from each other. As described above, since the second electrode 340 is formed as a single layer, it is easy to apply the ground signal to the second electrode 340.

The ultrasound probe 110 may further include an acoustic lens 350 for focusing the ultrasound wave. The acoustic lens 350 is disposed on the front surface of the piezoelectric layer 220 so as to focus the ultrasound wave generated by the piezoelectric layer 220. The acoustic lens 350 may be formed of a material such as silicon rubber having an acoustic impedance that is close to that of the object 10. Also, the acoustic lens 350 may have a convex center portion or a flat center portion. The acoustic lens 350 may be formed to have various shapes according to design of a designer.

The ultrasound probe 110 may further include a backing layer 360 for absorbing the ultrasound wave transmitted in a direction opposite to the object 10. The backing layer 360 supports the chip module substrate 320 at a rear surface of the chip module substrate 320, and may absorb the ultrasound wave that is transmitted to a rear portion of the piezoelectric layer 220 and is not directly used in the test or the diagnosis. In FIG. 3, the backing layer 360 is formed as an additional member separately from the chip module substrate 320, but is not limited thereto. That is, the substrate 240 in the chip module substrate 320 may be formed of a backing material so that the chip module substrate 320 performs as the backing layer 360.

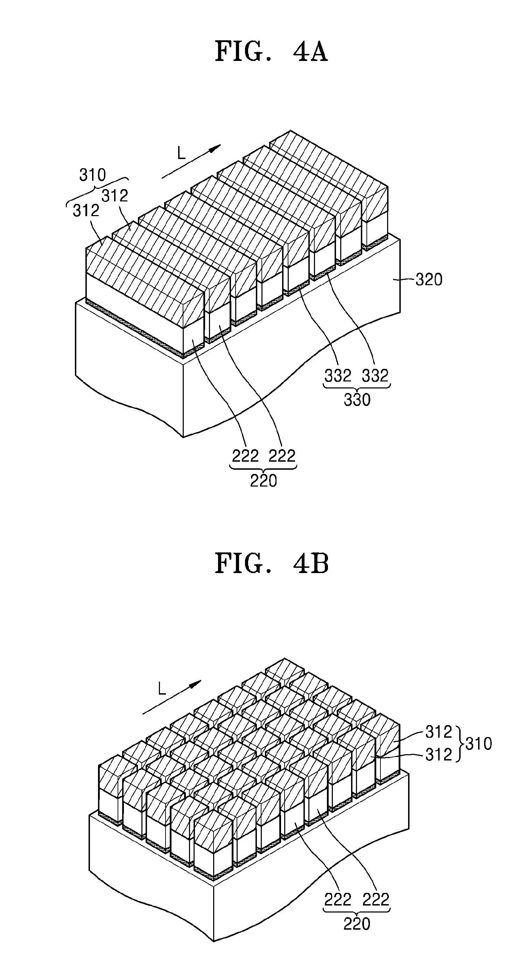

FIGS. 4A and 4B are diagrams showing arrangements of the piezoelectric members 222 in the piezoelectric layer 220 according to the embodiment of the inventive concept. As shown in FIG. 4A, the piezoelectric members 222 may be arranged one-dimensionally in a length direction L of the piezoelectric layer 220 on front surfaces of the electrode members 332. The above piezoelectric layer 220 may be referred to as a one-dimensional piezoelectric layer 220. The one-dimensional piezoelectric layer 220 may be a linear array or a curved array. The arrangement of the piezoelectric layer 220 may be variously set according to intention of the designer. The one-dimensional piezoelectric layer 220 is manufactured easily, thereby reducing manufacturing costs. However, it is difficult to realize a three-dimensional image by using the one-dimensional piezoelectric layer 220.

As shown in FIG. 4B, the piezoelectric members 222 may be arranged two-dimensionally in the length direction L of the piezoelectric layer 220 and a direction perpendicular to the length direction L. The above piezoelectric layer 220 may be referred to as a two-dimensional piezoelectric layer 220. The two-dimensional piezoelectric layer 220 may be a linear array or a curved array. The arrangement of the piezoelectric layer 220 may be variously set according to intention of the designer. Here, the two-dimensional piezoelectric layer 220 appropriately delays times of inputting signals to each of the piezoelectric members 222, and then, transmits the ultrasound waves to the object along with an external scan line for transmitting the ultrasound waves. Therefore, a 3D image may be obtained by using a plurality of echo signals. In addition, the more piezoelectric members 222, the clearer the ultrasound image.

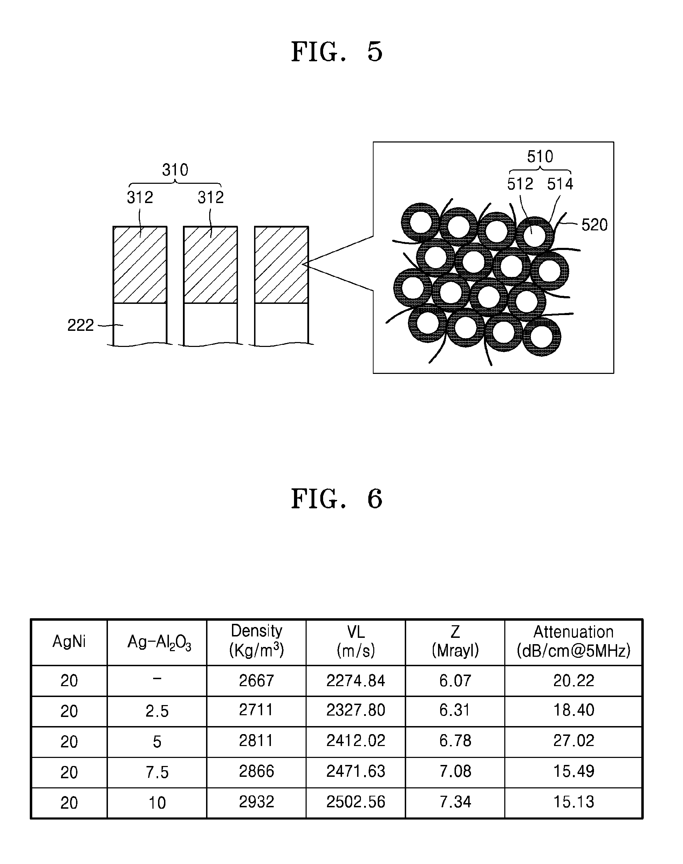

FIG. 5 is a diagram showing a structure of the matching layer 310 according to the exemplary embodiment. As shown in FIG. 5, the matching layer 310 may have a PIB structure, in which a plurality of conductive particles 510 and a binder 520 are mixed. Each of the plurality of conductive particles 510 may contact at least one neighboring conductive particle 510. Therefore, the matching layer 310 may have electric conductivity. The matching layer 310 may be also referred to as a matching member.

The conductive particles 510 in the matching layer 310 may have the identical sizes, or at least two of the conductive particles 510 may have different sizes. The conductive particles 510 in the matching layer 310 may be formed of the same material, or at least two of the conductive particles 510 may be formed of different materials. The electric conductivity and the acoustic impedance of the matching layer 310 may be determined according to a kind of the material forming the conductive particles 510, the sizes of the conductive particles 510, the content amount of the conductive particles 510, a material forming the binder 520, and the content amount of the binder 520.

Each of the plurality of conductive particles 510 includes a core 512 and a shield 514 formed of a material that is different from that of the core 512 and surrounding a surface of the core 512. Electric conductivity of the core 512 may be different from that of the shield 514.

The core 512 may have a lower electric conductivity than that of the shield 514. The core 512 may be formed of at least one selected from a non-conductive material, a semiconductor material, and a conductive material. Even if the core 512 is formed of a conductive material, the electric conductivity of the core 512 may be lower than that of the shield 514. For example, the core 512 may be formed of a conductive material such as copper (Cu), aluminum (Al), nickel (Ni), tungsten (W), beryllium (Be), indium (In), iron (Fe), lead (Pb), titanium (Ti), or tin (Sn). Otherwise, the core 512 may be formed of a non-conductive material such as glass, or a semiconductor material such as Si, germanium (Ge), or boron (B). The core 512 may be formed of metal oxide such as alumina (Al₂O₃), tin (SnO₂), hydrated iron (Fe₂O₃), or zinc oxide (ZnO). The metal oxide may belong to the conductive material, the semiconductor material, and the non-conductive material.

The shield 514 may have a greater electric conductivity than that of the core 512. For example, the shield 514 may be formed of at least one selected from silver (Ag), gold (Au), and platinum (Pt) that have high electric conductivity.

FIG. 5 shows the conductive particles 510 of spherical shapes, but one or more embodiments are not limited thereto. The conductive particles 510 may be formed as at least one selected from a flake, a bar, a rod, a wire, a fiber, and a conical shape. Hereinafter, it will be assumed that the conductive particle 510 has a spherical shape for convenience of description.

The binder 520 may fill spaces between the conductive particles 510 in the matching layer 310. The binder 520 may be a material for matching the acoustic impedance of the ultrasound wave generated from the piezoelectric layer 220 to an acoustic impedance of the object 10. For example, the binder 520 may include at least one selected from a polyvinyl butyral-based material, an acryl-based material, a polyester-based material, a phenoxy-based material, a polyvinyl formal-based material, a polyamide-based material, a polystyrene-based material, a polycarbonate-based material, a polyvinyl acetate-based material, a polyurethane-based material, an epoxy-based material, and a mixture thereof.

FIG. 6 is a table showing acoustic characteristic results of the matching layer 310 having the PIB structure according to the embodiment. The matching layer 310 is formed by differentiating a composition ratio between a first conductive particle including a core formed of Ni and a shield formed of Ag, and a second conductive particle including a core formed of Al₂O₃ and a shield formed of Ag. As shown in FIG. 6, the acoustic impedance Z of the matching layer 310 increases when the composition ratio of the second conductive particle increases.

The result shown in FIG. 6 represents that the acoustic impedance of the matching layer 310 may be adjusted by the composition ratio of the first and second conductive particles. For example, the composition ratio or the content amount of the first conductive particle increases from the piezoelectric layer 220 toward the acoustic lens 350 so as to reduce the acoustic impedance of the matching layer 310. The composition ratio or the content amount of the first conductive particle may be continuously or discontinuously changed.

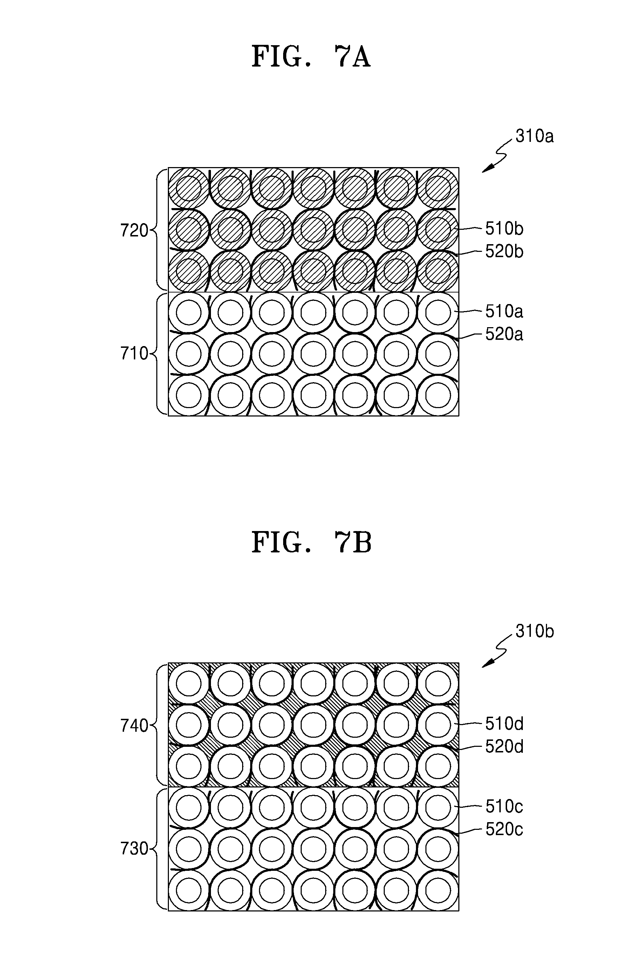

FIGS. 7A to 7D are diagrams showing modified examples of the matching layer 310 including the conductive particles 510 according to the embodiment of the inventive concept. The matching layer 310 may include a plurality of layers having different acoustic impedances from each other. Hereinafter, the matching layer 310 including two layers will be described below for convenience of description. The conductive particles and the binder will be described in order to increase an acoustic impedance of a lower layer to be greater than that of an upper layer.

As shown in FIG. 7A, a matching layer 310a may include a first matching layer 710 including first conductive particles 510a and a second matching layer 720 including second conductive particles 510b. Electric conductivity of the first conductive particle 510a may be different from that of the second conductive particle 510b. Since a material forming the first conductive particle 510a is different from a material forming the second conductive particle 510b, the acoustic impedance of the first conductive particle 510a may be different from that of the second conductive particle 510b. The acoustic impedance of the first conductive particle 510a may be greater than that of the second conductive particle 510b. For example, the acoustic impedance of a material forming a core included in the first conductive particle 510a may be greater than that of a material forming a core included in the second conductive particle 510b.

The acoustic impedance of a first binder 520a included in the first matching layer 710 may be the same as that of a second binder 520b included in the second matching layer. However, one or more embodiments of the inventive concept are not limited thereto. Even if the acoustic impedance of the first binder 520a is different that of the second binder 520b, the acoustic impedance of the first matching layer 710 according to combination of the first conductive particles 510a and the first binder 520a may be greater than that of the second matching layer 720 according to combination of the second conductive particles 510b and the second binder 520b.

Otherwise, as shown in FIG. 7B, the material of first conductive particles 510c included in a first matching layer 730 may be the same material as that of second conductive particles 510d included in a second matching layer 740. In addition, the material of a first binder 520c included in the first matching layer 730 may be different than that of a second binder 520d included in the second matching layer 740. The acoustic impedance of the first binder 520c may be greater than that of the second binder 520d.

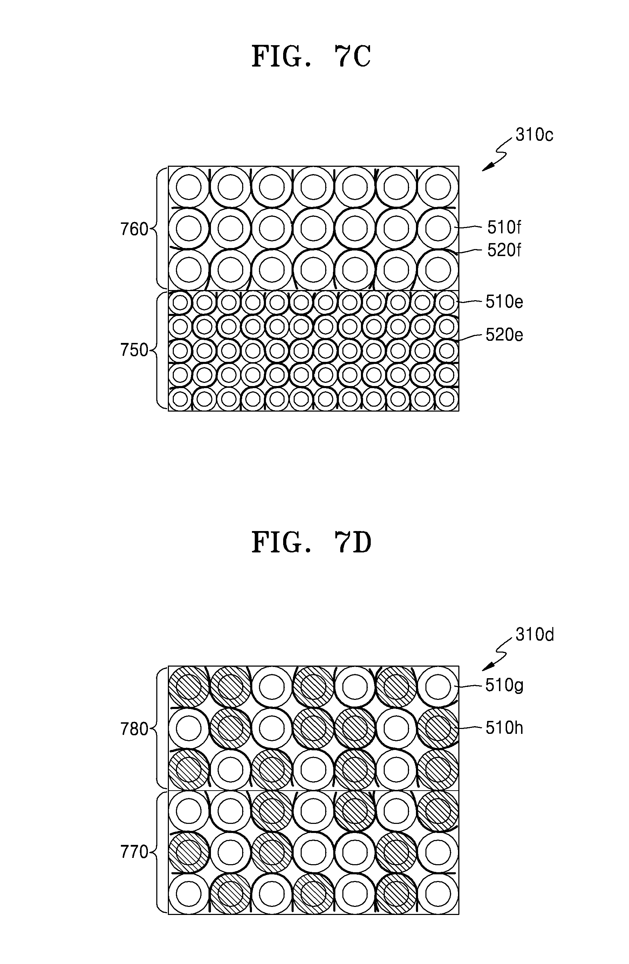

In addition, as shown in FIG. 7C, the acoustic impedance may be adjusted by a difference between sizes of first and second conductive particles 510e and 510f that are respectively included in first and second matching layers 750 and 760.

For example, even if the first and second conductive particles 510e and 510f are formed of the same material, the acoustic impedance may be increased when the content amount of the conductive particles 510 increases. Therefore, the first matching particles 510e are arranged in the first matching layer 750, and the second conductive particles 510f having larger sizes than those of the first conductive particles 510e may be arranged in the second matching layer 760.

The acoustic impedance of a first binder 520e included in the first matching layer 750 may be the same as that of a second binder 520f included in the second matching layer 760. However, one or more embodiments of the inventive concept are not limited thereto. The acoustic impedance of the first binders 520e may be different than that of the second binders 520f. Even if the acoustic impedance of the first binders 520e is different from that of the second binders 520f, the acoustic impedance of the first matching layer 750 according to the combination of the first conductive particles 510e and the first binder 520e may be greater than that of the second matching layer 760 according to combination of the second conductive particles 510f and the second binder 520f.

Otherwise, as shown in FIG. 7D, the acoustic impedance of a matching layer 310d may be adjusted according to the composition ratio or the content amounts of first and second conductive particles 510g and 510h. For example, the acoustic impedance of the first conductive particle 510g may be greater than that of the second conductive particle 510h by using at least one of the material and the size thereof. In the first matching layer 770, the content amount or the composition ratio of the first conductive particles 510g is greater than that of the second conductive particles 510h, and in the second matching layer 780, the content amount or the composition ratio of the first conductive particles 510g may be less than that of the second conductive particles 510h. Thus, the acoustic impedance of the first matching layer 770 may be greater than that of the second matching layer 780.

The method of adjusting the acoustic impedance by using the plurality of layers is described above. The layer having greater acoustic impedance is disposed at a side of the piezoelectric layer 220 and the layer having less acoustic impedance is disposed at a side of the acoustic lens 350, in the matching layer. In addition, a thickness direction of the matching layer may be a direction from the piezoelectric layer 220 to the acoustic lens 350 or from the acoustic lens 350 to the piezoelectric layer 220.

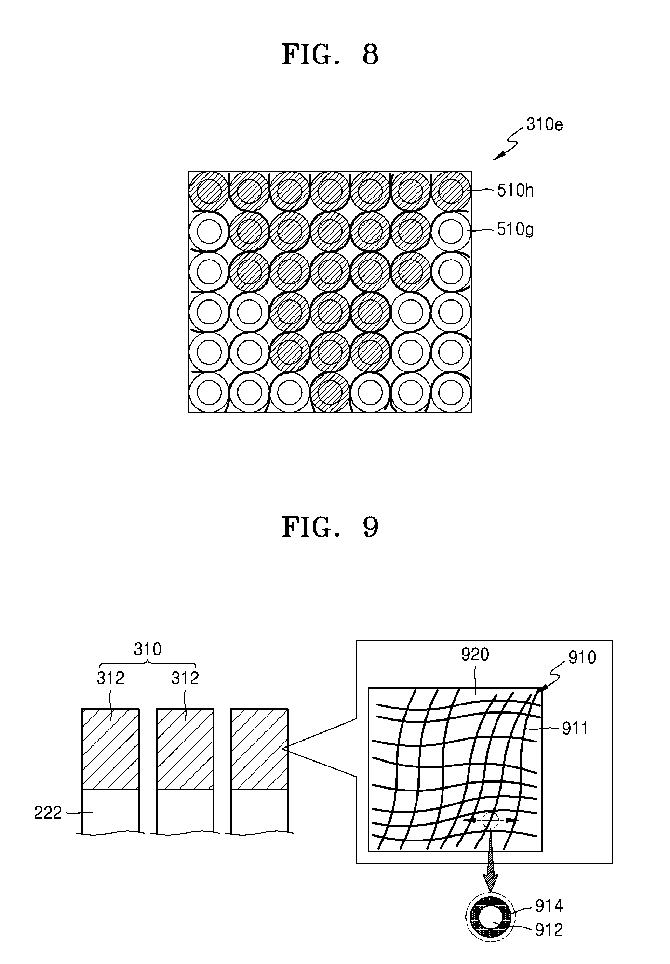

In addition, the acoustic impedance may be adjusted by using one single layer. FIG. 8 is a diagram of a matching layer 310e for adjusting the acoustic impedance by using a single layer. As shown in FIG. 8, the matching layer 310e may include first conductive particles 510g having greater acoustic impedance and second conductive particles 510h having smaller acoustic impedance. The acoustic impedances of the first and second conductive particles 510g and 510h may be determined according to materials forming the first and second conductive particles 510g and 510h and sizes of the first and second conductive particles 510g and 510h.

The composition ratios or the content amounts of the first conductive particles 510g and the second conductive particles 510h are uneven in the thickness direction of the matching layer 310e to adjust acoustic impedance. For example, the content amount or the composition ratio of the first conductive particles 510g gradually reduces and the content amount or the composition ratio of the second conductive particles 510h gradually increases from a lower portion to an upper portion of the matching layer 310e, so that the acoustic impedance of the lower portion in the matching layer 310e may be greater than that of the upper portion in the matching layer 310e. The content amount or the composition ratio may be continuously or discontinuously changed.

FIG. 9 is a diagram showing the matching layer 310 according to another embodiment. As shown in FIG. 9, the matching layer 310 may include a foam-type body 910 that is porous, and a filler 920 filling in the foam-type body 910. The foam-type body 910 may include a plurality of pores. In particular, the foam-type body 910 may include a plurality of pores and a plurality of bridges 911 connected to each other between the plurality of pores. The plurality of bridges 911 in the foam-type body 910 may be continuously connected. Thus, the matching layer 310 may have conductivity.

The plurality of bridges 911 may be connected to each other and may include a material having high electric conductivity. For example, the bridges 911 may be formed of at least one selected from Ag, Au, and Pt having high conductivity, but are not limited thereto.

Each of the bridges 911 may include a core 912 and a shield 914 formed of a different material than that of the core 912 and surrounding the core 912. The core 912 may have a lower electric conductivity than that of the shield 914. The core 912 may be formed of at least one of the non-conductive material, the semiconductor material, and the conductive material. Even if the core 912 is formed of a conductive material, the electric conductivity of the core 912 may be lower than that of the shield 914. For example, the core 912 may be formed of a conductive material such as Cu, Al, Ni, W, Be, In, Fe, Pb, Ti, or Sn. The core 912 may be formed of a non-conductive material such as glass or Si, or a semiconductor material. Also, the core 912 may be formed of metal oxide such as Si₂O₃. The metal oxide may be included in the conductive material, the semiconductor material, or the non-conductive material.

The foam-type body 910 may include a plurality of pores, at least some of which may overlap each other. That is, each of the plurality of pores may be connected to at least one neighboring pore. As such, an external material may be introduced into the foam-type body 910 through the pores. Sizes of the pores may not be necessarily constant.

The foam-type body 910 may be fabricated by using a bottom-up method, a method using a freeze-drying solution, or a method using a foam base. The above methods are examples of the method of manufacturing the foam-type body 910, and thus, one or more embodiments are not limited thereto.

The filler 920 may be filled in the plurality of pores. The filler 920 may be the same material as that of the binder 520 that is described above.

Since the foam-type body 910 includes the plurality of pores, the filler 920 may be filled in the plurality of pores. Also, the second electrode 340 and the piezoelectric layer 220 may be electrically connected to each other via the foam-type body 910. In addition, the acoustic impedance of the ultrasound wave generated from the piezoelectric layer 220 may be adjusted according to the kinds of materials forming the foam-type body 910 and the filler 920.

The ultrasound probe 110 according to the exemplary embodiment has the matching layer 310 having electric conductivity, and the ground signal may be applied to the piezoelectric layer 222 by using the second electrode 340 of a single layer. In addition, there is no need to perform a bonding process for attaching the piezoelectric layer 220 to the second electrode 340, and thus, the degree of freedom in designing structures of the ultrasound probe 110 may increase.

It should be understood that the exemplary embodiments described therein should be considered in a descriptive sense only and not for purposes of limitation. Descriptions of features or aspects within each embodiment should typically be considered as available for other similar features or aspects in other embodiments.

While one or more embodiments have been described with reference to the figures, it will be understood by those of ordinary skill in the art that various changes in form and details may be made therein without departing from the spirit and scope of the inventive concept as defined by the following claims.

## Claims

1. An ultrasound probe comprising:
a piezoelectric layer for converting an ultrasound wave to an electric signal or vice versa; and
a matching layer disposed on the piezoelectric layer and having a particle-in-binder (PIB) structure in which a plurality of conductive particles and a binder are mixed,
wherein at least one of the plurality of conductive particles comprises:
a core; and
a shield formed of a different material than that of the core and surrounding a surface of the core.

2. The ultrasound probe of claim 1, wherein an electric conductivity of the core is different from an electric conductivity of the shield.

3. The ultrasound probe of claim 2, wherein the electric conductivity of the shield is greater than the electric conductivity of the core.

4. The ultrasound probe of any one of claims 1 to 3, wherein the shield is formed of at least one selected from silver (Ag), gold (Au), and platinum (Pt).

5. The ultrasound probe of any one of claims 1 to 4, wherein the core is formed of at least one selected from a non-conductive material, a semiconductor material, and a conductive material.

6. The ultrasound probe of claim 5, wherein the core is formed of at least one selected from copper (Cu), aluminum (Al), nickel (Ni), tungsten (W), beryllium (Be), indium (In), iron (Fe), lead (Pb), titanium (Ti), tin (Sn), glass, and silicon (Si).

7. The ultrasound probe of any one of claims 1 to 6, wherein at least one of the plurality of conductive particles is formed as at least one selected from a sphere, a flake, a bar, a rod, a wire, a fiber, and a cone.

8. The ultrasound probe of any one of claims 1 to 7, wherein some neighboring conductive particles among the plurality of conductive particles at least partially overlap each other.

9. The ultrasound probe of any one of claims 1 to 8, wherein the plurality of conductive particles comprise a first conductive particle and a second conductive particle, wherein an electric conductivity of the first conductive particle is different from that of second conductive particle.

10. The ultrasound probe of claim 9, wherein the first conductive particle is different from the second conductive particle in at least one of a size and a material.

11. The ultrasound probe of claim 9 or 10, wherein a material forming a core of the first conductive particle is different from a material forming a core of the second conductive particle.

12. The ultrasound probe of any one of claims 9 to 11, wherein a composition ratio of the first conductive particle and the second conductive particle varies in a thickness direction of the matching layer.

13. The ultrasound probe of any one of claims 1 to 12, wherein a content amount of the plurality of conductive particles in the matching layer varies in a thickness direction of the matching layer.

14. The ultrasound probe of any one of claims 1 to 13, wherein the matching layer is grounded.

15. The ultrasound probe of any one of claims 1 to 14, further comprising a ground electrode contacting the matching layer.
